# EUROPEAN PATENT APPLICATION

(11) **EP 4 545 047 A1**
(43) Date of publication of application: **30.04.2025**
(21) Application number: 24207182.7
(22) Date of filing: 17.10.2024
(51) Int. Cl.: A61F 2/24, A61F 2/95, A61M 25/00

(54) **EXPANDABLE MESH SHEATH WITH PULL WIRES**

(30) Priority: 27.10.2023 US 202363593628 P; 10.10.2024 US 202418911435
(71) Applicant: Medtronic, Inc., Minneapolis, MN 55432 (US)
(72) Inventor: DUNLEA, Jake, Galway (IE); QUERO MARTIN, Andrea Quero, Galway (IE); FRANCIS, David, Claremorris (IE); CONERNEY, Mike Francis, Loughrea (IE)
(74) Representative: Zimmermann & Partner Patentanwälte mbB

(57) **Abstract**

An introducer apparatus is provided for delivering a heart valve prosthesis to a treatment site. The introducer apparatus includes a hub including a central passage and an engagement member. The introducer apparatus includes a sheath attached to the hub and including a first cross-sectional dimension. A plurality of wires extend between a first end and an opposing second end. The first end is attached to a distal sheath end of the sheath and the second end is attached to the engagement member. The engagement member moves the plurality of wires and the distal sheath end between an extended position, in which the sheath includes the first cross-sectional dimension, and a retracted position, in which the sheath includes a second cross-sectional dimension that is greater than the first cross-sectional dimension. Methods of delivering a heart valve prosthesis to a treatment site are provided.

## Description

### Cross-Reference to Related Applications

This application claims the benefit of U.S. Provisional Patent Application Serial No. 63/593,628, filed October 27, 2023, and U.S. Patent Application Serial No. 18/911,435, filed October 10, 2024, the entire contents of which are incorporated herein by reference.

### FIELD

The present disclosure relates generally to an expandable mesh sheath and, more particularly, to an expandable mesh sheath with one or more wires.

### BACKGROUND

It is known to provide a prosthetic heart valve assembly for implanting a heart valve prosthesis within a target site of the vasculature of a patient. The heart valve prosthesis can be moved from a radially-contracted position to a radially-expanded position.

### SUMMARY

The following presents a simplified summary of the disclosure to provide a basic understanding of some aspects described in the detailed description.

In aspects, an introducer apparatus is provided for delivering a heart valve prosthesis to a treatment site. The introducer apparatus comprises a hub comprising a central passage and an engagement member. The introducer apparatus comprises a sheath attached to the hub and comprising a first cross-sectional dimension along a plane that is perpendicular to a central axis of the sheath. The introducer apparatus comprises a plurality of wires extending between a first end and an opposing second end. The first end is attached to a distal sheath end of the sheath and the second end is attached to the engagement member. The engagement member is configured to move the plurality of wires and the distal sheath end between an extended position, in which the sheath comprises the first cross-sectional dimension, and a retracted position, in which the sheath comprises a second cross-sectional dimension that is greater than the first cross-sectional dimension.

In aspects, the engagement member is attached to a body portion of the hub, the engagement member configured to move relative to the body portion.

In aspects, the engagement member comprises a first engagement portion that is rotatable relative to the body portion, the first engagement portion circumferentially surrounding the central passage; and a second engagement portion received within the first engagement portion, the second engagement portion configured to move axially relative to the first engagement portion.

In aspects, the second end of the plurality of wires are attached to the second engagement portion such that the plurality of wires extend along the sheath.

In aspects, the engagement member circumferentially surrounds the body portion.

In aspects, the body portion defines at least one wire opening extending radially through the body portion between the central passage and an outer radial side of the body portion, the plurality of wires extending through the at least one wire opening such that the second end is attached to the engagement member.

In aspects, the engagement member is configured to rotate relative to the body portion, and wherein a locking mechanism contacts the engagement member and the body portion to selectively prevent rotation of the engagement member relative to the body portion.

In aspects, the engagement member comprises a first engagement projection and a second engagement projection that are spaced apart to define a first engagement channel between the first engagement projection and the second engagement projection.

In aspects, the body portion comprises a first body projection that is configured to be received within the first engagement channel such that the engagement member and the body portion are configured to move relative to one another between: a first position, in which the engagement member is spaced apart from the body portion such that the first body projection is not received within the first engagement channel, and at least one wire of the plurality of wires extends between the engagement member and the body portion; and a second position, in which the first body projection is received within the first engagement channel, and at least one wire of the plurality of wires extends within the first engagement channel.

In aspects, an introducer apparatus is provided for delivering a heart valve prosthesis to a treatment site. The introducer apparatus comprises a hub comprising a body portion surrounding a central passage, and an engagement member attached to the body portion and configured to move relative to the body portion. The introducer apparatus comprises a sheath attached to the hub. The sheath comprises a lumen in communication with the central passage. The sheath comprises a first cross-sectional dimension along a plane that is perpendicular to a central axis of the sheath. A plurality of wires extend between a first end and an opposing second end. The first end is attached to a distal sheath end of the sheath and the second end is attached to the engagement member. The engagement member is configured to move the plurality of wires and the distal sheath end from an extended position, in which the sheath comprises the first cross-sectional dimension and a first length, to a retracted position, in which the sheath comprises a second cross-sectional dimension, which is greater than the first cross-sectional dimension, and a second length, which is less than the first length.

In aspects, the engagement member comprises: a first engagement portion that is rotatable relative to the body portion, the first engagement portion circumferentially surrounding the central passage; and a second engagement portion received within the first engagement portion, the second engagement portion configured to move axially relative to the first engagement portion, the second end of the plurality of wires attached to the second engagement portion such that the plurality of wires extend along the sheath.

In aspects, the engagement member circumferentially surrounds the body portion and is configured to rotate relative to the body portion, and wherein the body portion defines at least one wire opening extending radially through the body portion between the central passage and an outer radial side of the body portion, the plurality of wires extending through the at least one wire opening such that the second end is attached to the engagement member.

In aspects, the engagement member comprises a first engagement projection and a second engagement projection that are spaced apart to define a first engagement channel between the first engagement projection and the second engagement projection, and wherein the body portion comprises a first body projection that is configured to be received within the first engagement channel, at least one wire of the plurality of wires extending between the engagement member and the body portion.

In aspects, methods of delivering a heart valve prosthesis to a treatment site comprise providing a sheath attached to a hub, the sheath comprising a first cross-sectional dimension along a plane that is perpendicular to a central axis of the sheath. Methods comprise moving a plurality of wires that are attached to a distal sheath end of the sheath in a proximal direction toward the hub. Methods comprise increasing a cross-sectional dimension of the sheath.

In aspects, moving the plurality of wires comprises rotating a first engagement portion relative to a body portion of the hub such that a second engagement portion, which is received within the first engagement portion, moves axially relative to the first engagement portion.

In aspects, the plurality of wires are attached to the second engagement portion such that the axial movement of the second engagement portion causes the plurality of wires and the distal sheath end of the sheath to move in the proximal direction.

In aspects, the plurality of wires extend through at least one wire opening of a body portion of the hub and are attached to an engagement member of the hub.

In aspects, moving the plurality of wires comprises rotating the engagement member relative to the body portion such that the plurality of wires and the distal sheath end of the sheath move axially in the proximal direction.

In aspects, at least one wire of the plurality of wires is positioned between an engagement member of the hub and a body portion of the hub, the at least one wire extending along a linear travel path.

In aspects, moving the plurality of wires comprises moving the engagement member and the body portion together to compress the at least one wire such that the at least one wire extends along a non-linear travel path.

Additional features and advantages of the aspects disclosed herein will be set forth in the detailed description that follows, and in part will be clear to those skilled in the art from that description or recognized by practicing the aspects described herein, including the detailed description which follows, the claims, as well as the appended drawings. It is to be understood that both the foregoing general description and the following detailed description present aspects intended to provide an overview or framework for understanding the nature and character of the aspects disclosed herein. The accompanying drawings are included to provide further understanding and are incorporated into and constitute a part of this specification. The drawings illustrate various aspects of the disclosure, and together with the description explain the principles and operations thereof.

### BRIEF DESCRIPTION OF THE DRAWINGS

These and other features, aspects and advantages are better understood when the following detailed description is read with reference to the accompanying drawings, in which:
**FIG. 1** schematically illustrates example aspects of a transcatheter heart valve prosthesis in accordance with aspects of the disclosure;
**FIG. 2** illustrates a top-down view of the transcatheter heart valve prosthesis in accordance with aspects of the disclosure;
**FIG. 3** illustrates a side view of a delivery assembly for delivering the transcatheter heart valve prosthesis in accordance with aspects of the disclosure;
**FIG. 4** illustrates a side view of the delivery assembly for delivering the transcatheter heart valve prosthesis in accordance with aspects of the disclosure;
**FIG. 5** illustrates an introducer apparatus in accordance with aspects of the disclosure;
**FIG. 6** illustrates an introducer apparatus in accordance with aspects of the disclosure;
**FIG. 7** schematically illustrates a side view of the transcatheter heart valve prosthesis positioned at a treatment site in accordance with aspects of the disclosure;
**FIG. 8** illustrates a side view of an introducer apparatus comprising a sheath in an extended position, in accordance with aspects of the disclosure;
**FIG. 9** illustrates a side view of the introducer apparatus comprising the sheath in a retracted position;
**FIG. 10** illustrates a side view of a first embodiment of the introducer apparatus, in accordance with aspects of the disclosure;
**FIG. 11** illustrates a sectional view similar to **FIG. 11** of the introducer apparatus, in accordance with aspects of the disclosure;
**FIG. 12** illustrates a sectional view of the introducer apparatus with the sheath in the extended position, in accordance with aspects of the disclosure;
**FIG. 13** illustrates a sectional view of the introducer apparatus with the sheath in the retracted position, in accordance with aspects of the disclosure;
**FIG. 14** illustrates a side view of a second embodiment of the introducer apparatus, in accordance with aspects of the disclosure;
**FIG. 15** illustrates a sectional view similar to **FIG. 14** of the introducer apparatus, in accordance with aspects of the disclosure;
**FIG. 16** illustrates a sectional view similar to **FIG. 15** of the introducer apparatus, in accordance with aspects of the disclosure;
**FIG. 17** illustrates a sectional view of the introducer apparatus with the sheath in the extended position, in accordance with aspects of the disclosure;
**FIG. 18** illustrates a sectional view of the introducer apparatus with the sheath in the retracted position, in accordance with aspects of the disclosure;
**FIG. 19** illustrates a side view of a third embodiment of the introducer apparatus, in accordance with aspects of the disclosure;
**FIG. 20** illustrates a side view of the introducer apparatus similar to **FIG. 19**, in accordance with aspects of the disclosure; and
**FIG. 21** illustrates a side view of a fourth embodiment of the introducer apparatus, in accordance with aspects of the disclosure.

### DETAILED DESCRIPTION

Aspects will now be described more fully hereinafter with reference to the accompanying drawings in which example aspects are shown. Whenever possible, the same reference numerals are used throughout the drawings to refer to the same or like parts. However, this disclosure may be embodied in many different forms and should not be construed as limited to the aspects set forth herein.

As used herein, the term "about" means that amounts, sizes, formulations, parameters, and other quantities and characteristics are not, and need not be, exact, but may be approximate and/or larger or smaller, as desired, reflecting tolerances, conversion factors, rounding off, measurement error and the like, and other factors known to those of skill in the art.

Ranges can be expressed herein as from "about" one value, and/or to "about" another value. When such a range is expressed, aspects include from the one value to the other value. Similarly, when values are expressed as approximations by use of the antecedent "about," it will be understood that the value forms another aspect. It will be further understood that the endpoints of each of the ranges are significant both in relation to the other endpoint, and independently of the other endpoint.

Directional terms as used herein - for example up, down, right, left, front, back, top, bottom, upper, lower, etc. - are made only with reference to the figures as drawn and are not intended to imply absolute orientation.

Unless otherwise expressly stated, it is in no way intended that any methods set forth herein be construed as requiring that its steps be performed in a specific order, nor that with any apparatus, specific orientations be required. Accordingly, where a method claim does not actually recite an order to be followed by its steps, or that any apparatus claim does not actually recite an order or orientation to individual components, or it is not otherwise specifically stated in the claims or description that the steps are to be limited to a specific order, or that a specific order or orientation to components of an apparatus is not recited, it is in no way intended that an order or orientation be inferred in any respect. This holds for any possible non-express basis for interpretation, including matters of logic relative to arrangement of steps, operational flow, order of components, or orientation of components; plain meaning derived from grammatical organization or punctuation, and; the number or type of aspects described in the specification.

As used herein, the singular forms "a," "an," and "the" include plural references unless the context clearly dictates otherwise. Thus, for example, reference to "a" component includes aspects having two or more such components, unless the context clearly indicates otherwise.

The word "exemplary," "example," or various forms thereof are used herein to mean serving as an example, instance, or illustration. Any aspect or design described herein as "exemplary" or as an "example" should not be construed as preferred or advantageous over other aspects or designs. Furthermore, examples are provided solely for purposes of clarity and understanding and are not meant to limit or restrict the disclosed subject matter or relevant portions of this disclosure in any manner. It can be appreciated that a myriad of additional or alternate examples of varying scope could have been presented but have been omitted for purposes of brevity.

As used herein, the terms "comprising," "including," and variations thereof shall be construed as synonymous and open-ended, unless otherwise indicated. A list of elements following the transitional phrases comprising or including is a non-exclusive list, such that elements in addition to those specifically recited in the list may also be present.

The terms "substantial," "substantially," and variations thereof as used herein are intended to represent that a described feature is equal or approximately equal to a value or description. For example, a "substantially planar" surface is intended to denote a surface that is planar or approximately planar. Moreover, "substantially" is intended to denote that two values are equal or approximately equal. The term "substantially" may denote values within about 10% of each other, for example, within about 5% of each other, or within about 2% of each other.

Modifications may be made to the instant disclosure without departing from the scope or spirit of the claimed subject matter. Unless specified otherwise, "first," "second," or the like are not intended to imply a temporal aspect, a spatial aspect, an ordering, etc. Rather, such terms are merely used as identifiers, names, etc. for features, elements, items, etc. For example, a first end and a second end generally correspond to end A and end B or two different ends.

Unless otherwise indicated, the terms "distal" and "proximal" are used in the following description with respect to a position or direction relative to the treating clinician. "Distal" and "distally" are positions distant from or in a direction away from the clinician, and "proximal" and "proximally" are positions near or in a direction toward the clinician. In addition, the term "self-expanding" may be used in the following description with reference to one or more valve or stent structures of the prostheses hereof and is intended to convey that the structures are shaped or formed from a material that can be provided with a mechanical memory to return the structure from a compressed or constricted delivery configuration to an expanded deployed configuration or vice versa. Non-exhaustive exemplary self-expanding materials include stainless steel, a pseudo-elastic metal such as a nickel titanium alloy or nitinol, various polymers, or a so-called super alloy, which may have a hub metal of nickel, cobalt, chromium, or other metal. Mechanical memory may be imparted to a wire or stent structure by thermal treatment to achieve a spring temper in stainless steel, for example, or to set a shape memory in a susceptible metal alloy, such as nitinol. Various polymers that can be made to have shape memory characteristics may also be suitable for use in aspects hereof to include polymers such as polynorborene, trans-polyisoprene, styrene-butadiene, and polyurethane. As well poly L-D lactic copolymer, oligo caprylactone copolymer and poly cyclo-octine can be used separately or in conjunction with other shape memory polymers.

Diseases associated with heart valves, such as those caused by damage or a defect, can include stenosis and valvular insufficiency or regurgitation. For example, valvular stenosis causes the valve to become narrowed and hardened which can prevent blood flow to a downstream heart chamber from occurring at the proper flow rate and may cause the heart to work harder to pump the blood through the diseased valve. Valvular insufficiency or regurgitation occurs when the valve does not close completely, allowing blood to flow backwards, thereby causing the heart to be less efficient. A diseased or damaged valve, which can be congenital, age-related, drug-induced, or in some instances, caused by infection, can result in an enlarged, thickened heart that loses elasticity and efficiency. Some symptoms of heart valve diseases can include weakness, shortness of breath, dizziness, fainting, palpitations, anemia and edema, and blood clots which can increase the likelihood of stroke or pulmonary embolism. Symptoms can often be severe enough to be debilitating and/or life threatening.

Heart valve prostheses have been developed for repair and replacement of diseased and/or damaged heart valves. Such heart valve prostheses can be percutaneously delivered and deployed at the site of the diseased heart valve through catheter-hub delivery systems. Such heart valve prostheses generally include a frame or stent and a prosthetic valve mounted within the frame. Such heart valve prostheses are delivered in a radially compressed or crimped configuration so that the heart valve prosthesis can be advanced through the patient's vasculature. Once positioned at the treatment site, the heart valve prosthesis is expanded to engage tissue at the diseased heart valve region to, for instance, hold the heart valve prosthesis in position.

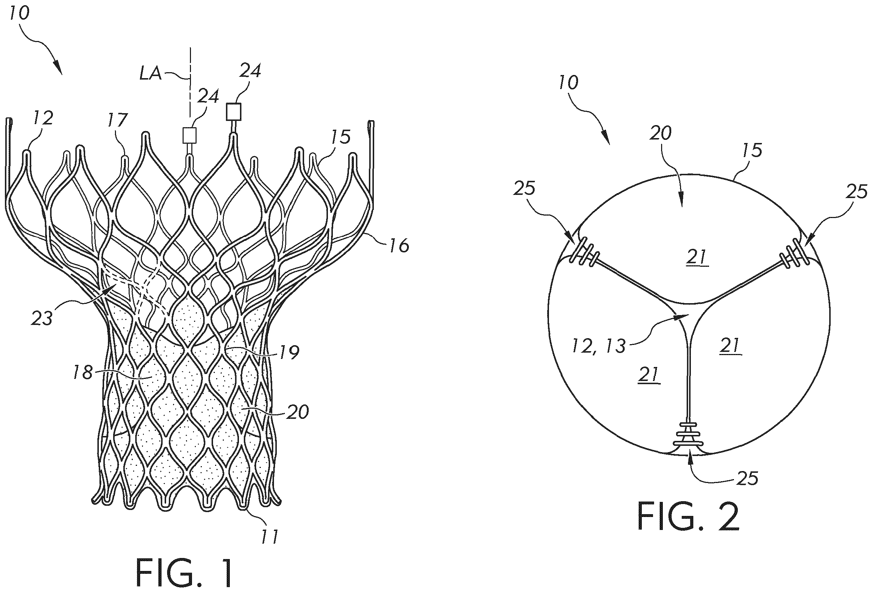

**FIGS. 1** and **2** illustrate an example transcatheter heart valve prosthesis **10.** The delivery assemblies described herein may be used with the transcatheter heart valve prosthesis **10** and/or other transcatheter heart valve prostheses. The transcatheter heart valve prosthesis **10** is illustrated to facilitate description of the disclosure. The following description of the transcatheter heart valve prosthesis **10** is merely exemplary in nature and is not intended to limit the invention or the application and uses of the invention.

**FIGS. 1** and **2** illustrate a side view and a top/end view, respectively, of the transcatheter heart valve prosthesis **10.** The transcatheter heart valve prosthesis **10** includes a radially-expandable frame or stent **15** and a prosthetic valve **20.** The frame **15** of the transcatheter heart valve prosthesis **10** supports the prosthetic valve **20** within an interior of the frame **15.** In the example transcatheter heart valve prosthesis **10** shown in **FIGS. 1** and **2**, the frame **15** is self-expandable. However, this is not meant to be limiting, and the frame **15** can be balloon-expandable or mechanically expandable in other embodiments. In some embodiments, the transcatheter heart valve prosthesis **10** may be delivered to and implanted at a treatment site within a patient to replace any of an aortic valve, a pulmonic valve, a mitral valve, and a tricuspid valve. The valve to be replaced may be a native valve or a previously-implanted prosthetic valve, such as a failed surgical replacement valve or a failed transcatheter valve.

The prosthetic valve **20** includes at least one leaflet **21** disposed within and secured to the frame **15.** In the embodiment shown in **FIGS. 1** and **2**, the prosthetic valve **20** includes exactly three leaflets **21**, as shown in **FIG. 2****.** However, this is not meant to be limiting, as the prosthetic valve **20** may include more or fewer leaflets **21.** The valve leaflets **21** open and close to regulate flow through the transcatheter heart valve prosthesis **10.**

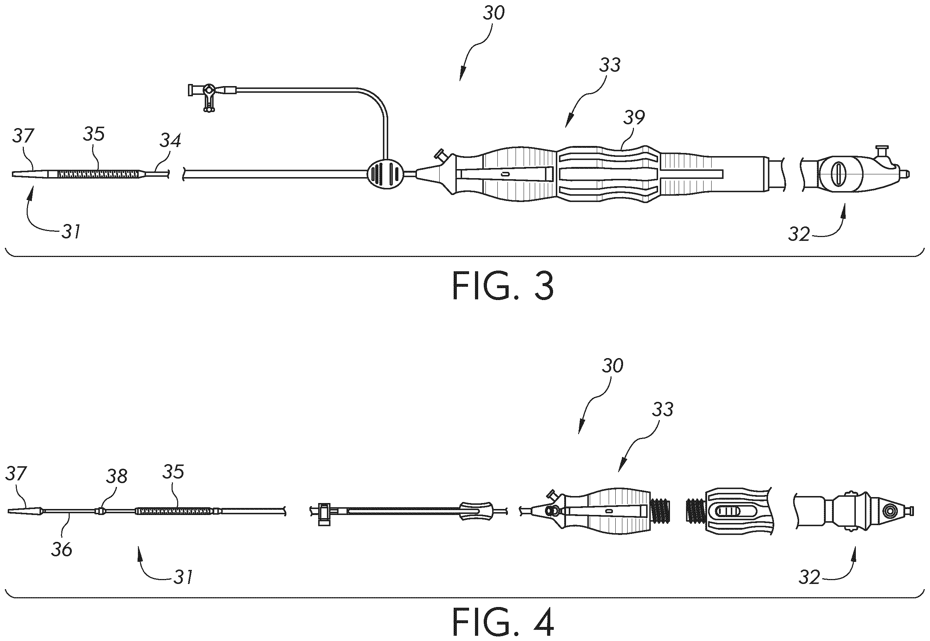

As shown in **FIG. 1**, the transcatheter heart valve prosthesis **10** includes an inflow end **11** and an outflow end **12.** The prosthetic leaflets **21** are attached to the frame **15** at commissures **25** such that when pressure at the inflow end **11** exceeds pressure at the outflow end **12**, the prosthetic leaflets **21** open to allow blood flow through the heart valve prosthesis **10** from the inflow end **11** to the outflow end **12.** When the pressure at the outflow end **12** exceeds pressure at the inflow end **11**, the prosthetic leaflets **21** close to prevent blood flow from the outflow end **12** to the inflow end **11.** Accordingly, the at least one leaflet (e.g., the prosthetic leaflets **21**) can be attached to the plurality of struts **16**, for example, by being directly attached to the plurality of struts **16** at the commissures **25**, or by being indirectly attached to the plurality of struts **16**, for example, by being attached to a skirt, a commissure bracket, or other structure (e.g., mechanical actuator) that is attached to the plurality of struts **16.** In aspects, the heart valve prosthesis **10** can comprise one or more attachment members **24** (e.g., paddles) positioned at an end, for example, the outflow end **12.** The attachment members **24** can be received within pockets of a spindle **38** (e.g., illustrated in **FIG. 4**), such that the spindle **38** and the attachment members **24** can interact to facilitate loading of the transcatheter heart valve prosthesis **10** and, in aspects, allow for possible recapture of the transcatheter heart valve prosthesis **10** during the deployment process.

The frame **15** of the transcatheter heart valve prosthesis **10** further includes a plurality of struts **16** that are arranged to form a plurality of openings or cells **18** arranged circumferentially around a longitudinal axis LA of the transcatheter heart valve prosthesis **10** and longitudinally to form a tubular structure defining a central lumen of the transcatheter heart valve prosthesis **10.** For example, the frame **15** can extend along the longitudinal axis LA between the inflow end **11** and the outflow end **12.** The frame **15** is configured to secure the prosthetic valve **20** within the central lumen of the frame **15** and to secure the transcatheter heart valve prosthesis **10** in place in the vasculature of the patient. The struts **16** are defined herein as the elongated wire segments of the frame **15.** Struts **16** come together to form crowns **17** or nodes **19**, as can be seen in **FIG. 1****.** The frame **15** of the heart valve prosthesis **10** includes a plurality of cells **18** defined as the spaces between the plurality of crowns **17**, the plurality of nodes **19**, and the plurality of struts **16.** The frame **15**, and, thus, the plurality of struts **16**, can be adjustable between a radially-collapsed position and a radially-expanded position.

In the example embodiment shown in **FIG. 1**, the plurality of cells **18** may be diamond-shaped. In the example embodiment shown, the plurality of cells include a plurality of first cells **18** and, in aspects, access cells (e.g., an access cell **23**). In particular, the access cells may be larger than the first cells **18** and can provide access to one or more coronary arteries when the transcatheter heart valve prosthesis **10** is implanted in the patient. **FIG. 1** illustrates an example of an access cell **23**, with the struts **16** at the access cell **23** illustrated with dashed lines to show that the struts **16** may not be present at the access cell **23**, thus allowing for the access cell **23** to be larger than the first cells **18.** The access cells can have an enlarged area relative or compared to the first cells **18.** In some embodiments the transcatheter heart valve prosthesis **10** may include an outer skirt extending circumferentially around an outer circumference of the stent **15** at or near the inflow end **11** to prevent paravalvular leakage of blood around the outside of the transcatheter heart valve prosthesis **10** once implanted in the patient.

**FIGS. 3** and **4** show schematically side views of a transcatheter heart valve delivery assembly **30** (e.g., "delivery assembly") for delivering and deploying a transcatheter heart valve prosthesis (e.g., transcatheter heart valve prosthesis **10**) according to embodiments hereof. One skilled in the art will realize that **FIGS. 3** and **4** illustrate one example of a delivery assembly **30** and that components illustrated in **FIGS. 3** and **4** may be removed and/or additional components may be added. The delivery assembly **30** includes a distal end **31**, a proximal end **32**, and a handle **33.** The handle **33** enables a physician to manipulate a distal portion of the delivery assembly **30** and includes actuators for moving parts of the delivery assembly **30** relative to other parts. In the delivery assembly **30**, an outer shaft **34** is coupled to an actuator **39** of the handle **33** for moving the outer shaft **34** relative to an inner shaft **36.**

A distal portion of the outer shaft **34**, referred to as a capsule **35**, is configured to surround a transcatheter heart valve prosthesis (e.g., transcatheter heart valve prosthesis **10**) during delivery to the treatment site (e.g., a native heart valve) and is retracted from the transcatheter heart valve prosthesis to expose the transcatheter heart valve prosthesis such that it self-expands (in self-expanding embodiments). In this way, the capsule **35** is in frictional engagement with the heart valve prosthesis **10.** The inner shaft **36** can be coupled to the handle **33** (e.g., by being directly connected and in contact with the handle **33**, or by being indirectly connected to the handle **33** with intermediate structures between the inner shaft **36** and the handle **33**) and movement of the handle **33** can translate to movement of the inner shaft **36** and a distal tip or nosecone **37** coupled to a distal end of the inner shaft **36**. The inner shaft **36** and distal tip or nosecone **37** may also be translated relative to the outer shaft **34** and the handle **33** via a tip retractor. In the embodiment shown, the inner shaft **36** includes a retainer or spindle **38** for receiving the paddles (e.g., attachment members **24**) of the transcatheter heart valve prosthesis **10.**

When the actuator **39** is actuated, the actuator **39** moves the outer shaft **34** and the capsule **35** relative to the inner shaft **36,** as shown in **FIG. 4****.** As known to those skilled in the art, when the delivery assembly **30** is in position such that the transcatheter heart valve prosthesis **10** is at the desired position at the treatment site in the patient's vasculature, the actuator **39** is actuated (e.g., rotated) to move the capsule **35** relative to the inner shaft **36** and the transcatheter heart valve prosthesis **10** disposed between the inner shaft **36** and the capsule **35,** thereby enabling the transcatheter heart valve prosthesis **10** to deploy via self-expansion at the treatment site and release from the retainer **38,** as shown in **FIG. 4** (without showing the transcatheter heart valve prosthesis **10**).

Minimally invasive percutaneous interventional procedures, including endovascular procedures, require access to the venous or arterial system. In general, it is desirable to make the smallest incision point with the shortest tissue contact time when entering the body. Small incisions and short tissue contact time generally lead to improved patient outcomes, less complications, and less trauma to the vessels or organs being accessed, as well as less trauma to the skin and tissue through which the access point is created. Access is required for various medical procedures that deliver or implant structural elements (such as heart valves, heart valve repair devices, occluders, grafts, electrical stimulators, leads, etc.) percutaneously. Some procedures employ relatively large devices that require relatively large sheaths to deliver the devices to the intended site within the body. With such procedures, access site trauma can occur, often resulting in vessel damage, excessive bleeding, increased case time, increased risk of infection, and increased hospitalization time. To reduce access trauma, physicians try to use the smallest devices possible and place the smallest sheath size. This can be problematic, however, if during the procedure the physician discovers a larger device is needed. This leads to a need to upsize the sheath, which is a lengthy procedure and leads to increased risk to the patient. Expandable sheaths can be expanded within the body and thus do not require removal to upsize.

Expandable sheath designs may be regionally or locally expansive to selectively and temporarily expand when the device is passing through a region of the sheath and to retract or recover when the device is not passing or has already passed through the sheath. Embodiments disclosed herein may be employed with an expandable introducer sheath that may solve these and other issues that contribute to vascular trauma. The expandable introducer sheath is described with respect to percutaneous access for transcatheter heart valve repair or replacement, and it should be understood that one or more features of the expandable introducer sheath may be employed alone or in combination for other medical procedures requiring percutaneous access, including but not limited to placement of stents, angioplasty, removal of arterial or venous calcification, and pre-dilatation or post-dilatation.

Various embodiments disclosed herein may include an introducer sheath that has a selectively expandable diameter to allow for the passage of a relatively larger device therethrough and further is configured to return to its original diameter upon passage of the device. The various embodiments may reduce damage to surrounding tissues by reducing contact with those tissues and by eliminating the need to exchange sheaths of different sizes. As a result, these embodiments can reduce procedure time, vascular trauma, bleeding, and the resulting risk of infection and other complications.

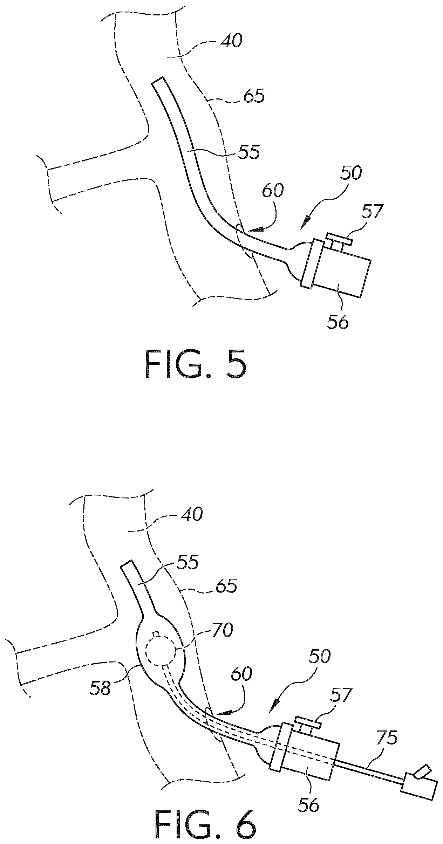

**FIGS. 5** and **6** depict one embodiment of an introducer apparatus **50** positioned through an incision **60** in the skin **65** of a patient and into a vessel **40** of a patient. The introducer apparatus **50** has a tubular sheath **55** and a proximal hub **56** with a hemostatic seal and a luer lock **57.** **FIG. 5** shows the sheath **55** of the introducer apparatus **50** positioned in the vessel **40** in its normal, unexpanded state, while **FIG. 6** shows the introducer apparatus **50** positioned in the vessel **40** with a delivery device **75** delivering another device **70** that is being advanced through the introducer apparatus **50** such that the tubular sheath **55** expands or deforms at the location where the device **70** is passing through. The sheath **55** expands at expanded region **58** when the device **70** passes through and then retracts or recovers to its original diameter after the device **70** moves past or is removed from the sheath **55.** Thus, the tubular sheath **55** is configured to be expandable and retractable.

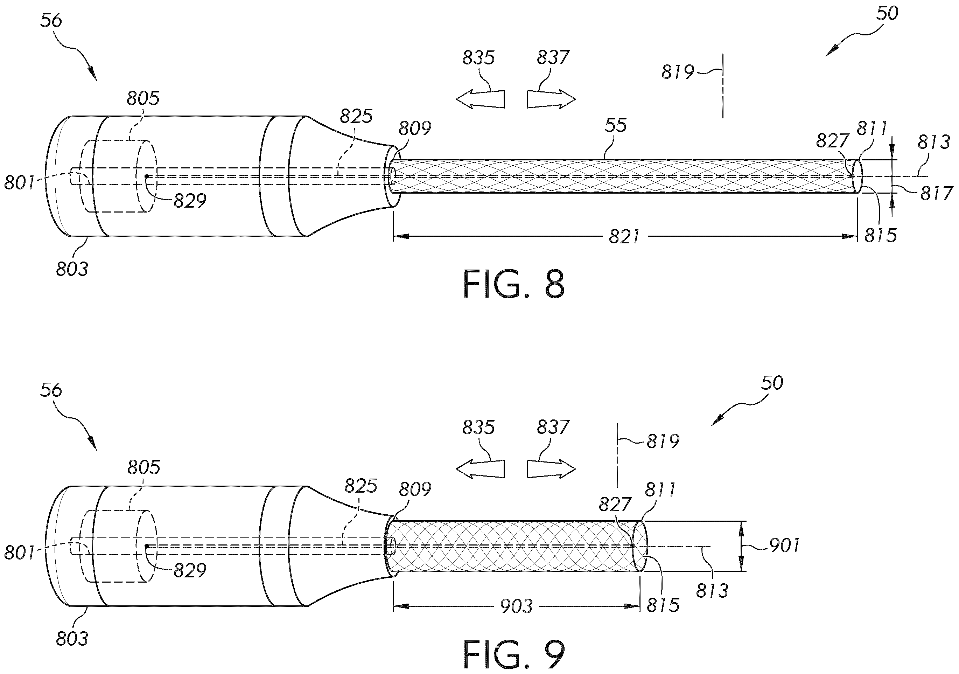

In certain embodiments, the expandability of the sheath **55** (and any sheath described according to any embodiment set forth herein) is achieved via the elasticity of the sheath **55**, which can result in the sheath **55** being either self-expandable or self-expanding or mechanically expandable or mechanically expanding. For purposes of this application, self-expandable means that the sheath **55** is configured to expand to a predetermined or nominal diameter automatically (without any type of actuation, mechanical or otherwise). Further, for purposes of this application, mechanically expandable means that the sheath **55** is configured to expand when a positionable medical device is positioned through the sheath **55.** That is, the device itself that is being passed through the sheath **55** causes the expansion of the sheath **55,** as depicted in **FIG. 6****.** Alternatively, and as described below relative to **FIGS. 8-21**, the expandable characteristics of the sheath **55** can be caused by one or more wires that move the sheath **55** between an expanded position with a smaller diameter and a retracted position with a larger diameter.

After passage of the device, the sheath **55** is configured to be contractable, retractable, or recoverable to its original, unexpanded state as depicted in **FIG. 5****.** The retractability can be, in certain embodiments, achieved by the elasticity of the sheath **55,** which can result in the sheath **55** being either self-retractable or self-retracting, self-recoverable, or self-contractable, or mechanically retractable or mechanically retracting, mechanically recoverable, or mechanically contractable. For purposes of this application, self-retractable means that the sheath **55** is configured to retract to a predetermined or nominal diameter automatically (without any type of actuation, mechanical or otherwise). Further, for purposes of this application, mechanically retractable means that the sheath **55** is configured to retract when a device or component is used to cause the sheath **55** to retract or recover. Alternatively, the retractable characteristics of the sheath **55** can be caused by something other than elasticity.

For purposes of this application, any device that can be positioned through an introducer sheath according to any embodiment disclosed or contemplated herein can be referred to as a positionable medical device or insertable medical device. Such devices include guidewires, dilators, delivery devices (for delivery and/or placement of structural elements such as heart valves, heart valve repair devices, occluders, grafts, electrical stimulators, leads, etc.), guide catheters, guiding sheaths, diagnostic catheters, stent delivery systems, balloon catheters, and other known vascular devices. Other devices can include non-vascular devices such as scopes and other common surgical instruments. Further, the introducer sheath is configured to receive tissues or organs. Thus, as one nonlimiting example, the introducer sheath **55** is described as being an expandable introducer sheath **55** for introduction of a delivery assembly **30** including a transcatheter heart valve prosthesis **10.**

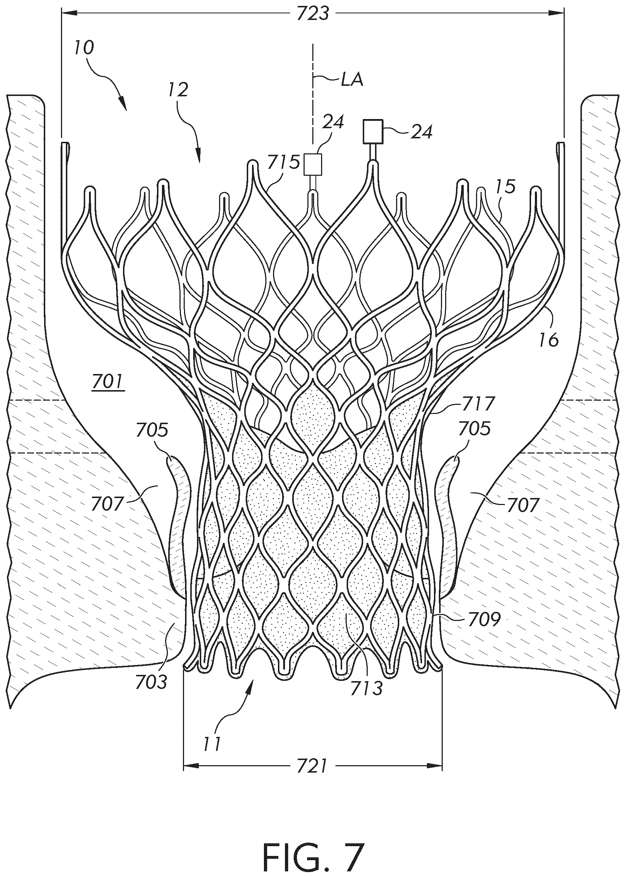

**FIG. 7** illustrates the heart valve prosthesis **10** at a treatment site **701** within a patient's vasculature. In aspects, the treatment site **701** can comprise a location of a native aortic annulus (hereinafter "annulus") **703** of a native heart valve, for example, the annulus of a patient's left ventricle. The treatment site **701** can comprise one or more native valve leaflets **705** and corresponding native sinuses **707.** In some instances, paravalvular leakage can occur when blood travels through a gap **709** around the outside of the transcatheter heart valve prosthesis **10,** with the gap **709** formed between the transcatheter heart valve prosthesis **10** and the annulus **703.** To avoid paravalvular leakage, the heart valve prosthesis **10** can be radially expanded such that an outer radial surface of the heart valve prosthesis **10** can contact the annulus **703** and/or the native valve leaflets **705,** thus reducing or eliminating the gap **709** and causing the blood to flow through the central lumen **13** of the heart valve prosthesis **10.** The frame **15** of the heart valve prosthesis **10** can comprise an asymmetric hourglass shape with a first section **713** at the inflow end **11,** a second section **715** at the outflow end **12,** and a waist section **717** positioned between the first section **713** and the second section **715.** In aspects, the first section **713** can comprise a first diameter **721** and the second section **715** can comprise a second diameter **723,** with the second diameter **723** greater than the first diameter **721.** Additionally, in some embodiments the transcatheter heart valve prosthesis **10** may include an outer skirt extending circumferentially around an outer circumference of the frame **15** at or near the inflow end **11** to prevent paravalvular leakage of blood around the outside of the transcatheter heart valve prosthesis **10** once implanted in the patient. Thus, features of the disclosure may be employed alone or in combination with a heart valve prosthesis **10** having an outer skirt or other external sealing member (not shown) or a heart valve prosthesis **10** having no outer skirt.

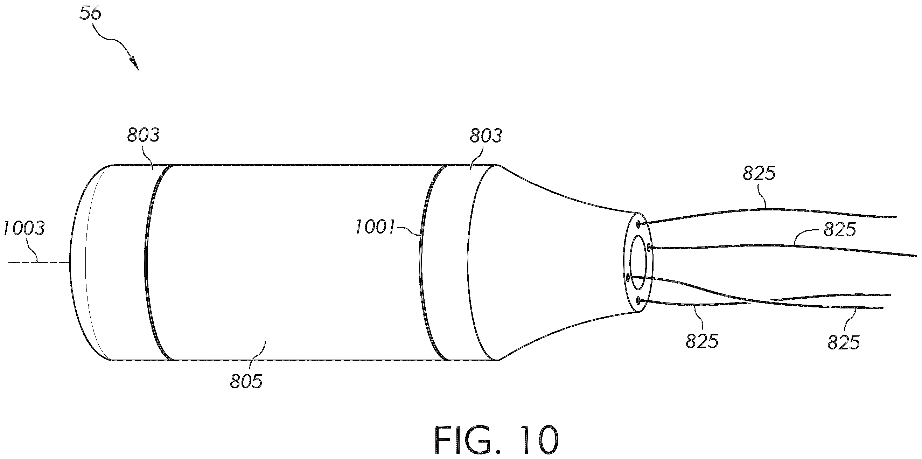

**FIGS. 8-9** illustrate a side view of the introducer apparatus **50** comprising the sheath **55** and the hub **56.** The hub **56** can comprise a central passage **801** that is substantially hollow and through which one or more structures can pass. The hub **56** can comprise a body portion **803** and an engagement member **805.** The body portion **803** can circumferentially surround the central passage **801** such that one or more walls of the body portion **803** can at least partially define the central passage **801.** Portions of the hub **56,** for example, the central passage **801,** the body portion **803,** and the engagement member **805,** are illustrated schematically in **FIGS. 8-9** due to the central passage **801,** the body portion **803,** and the engagement member **805** comprising several different embodiments illustrated in **FIGS. 10-21****.** As such, **FIG. 8** generally illustrates a position of the sheath **55** relative to the hub **56,** wherein the sheath **55** is in an expanded position. **FIG. 9** generally illustrates movement of the sheath **55** relative to the hub **56** such that the sheath **55** is in a retracted position. However, the position and structure of the engagement member **805** relative to the body portion **803** is not intended to be limited to the examples illustrated in **FIGS. 8-9****,** as the engagement member **805** is illustrated schematically. Rather, the embodiments of **FIGS. 10-21** illustrate other examples of the position and structure of the engagement member **805.**

The sheath **55** can extend between a proximal sheath end **809** and a distal sheath end **811** along a central axis **813.** The sheath **55** can be attached to the hub **56,** for example, with the proximal sheath end **809** attached to the hub **56.** The sheath **55** can comprise a lumen **815** (e.g., elongated passageway) that extends along the length of the sheath **55** between the proximal sheath end **809** and the distal sheath end **811.** In this way, the lumen **815** may be in communication with the central passage **801.** By being in communication, the lumen **815** and the central passage **801** can be oriented relative to one another such that an object (e.g., the delivery device **75,** the heart valve prosthesis **10,** etc.) can pass from the central passage **801** and into the lumen **815** and vice versa. Though not required, in aspects, the lumen **815** and the central passage **801** can be positioned in an end-to-end orientation.

The sheath **55** can comprise a first cross-sectional dimension **817** along a plane **819** that is perpendicular to the central axis **813.** In aspects, the sheath **55** can comprise a substantially circular cross-sectional shape, such that the first cross-sectional dimension **817** comprises a first diameter of the sheath **55.** The sheath **55** can comprise a first length **821** that is measured along the central axis **813** between the hub **56** and the distal sheath end **811.** In aspects, the sheath **55** can be biased toward the first cross-sectional dimension **817** and the first length **821** such that, in the absence of forces (e.g., compressive, tensile, etc.) acting upon the sheath **55,** the sheath **55** comprises the first cross-sectional dimension **817** and the first length **821.** However, as described herein, the first cross-sectional dimension **817** and the first length **821** can be changed due to forces acting upon the sheath **55.** Methods can therefore comprise providing the sheath **55** attached to the hub **56,** with the sheath **55** comprising the first cross-sectional dimension **817** along the plane **819** that is perpendicular to the central axis **813.**

The introducer apparatus **50** can comprise a plurality of wires **825** extending between a first end **827** and an opposing second end **829.** The first end **827** can be attached to the distal sheath end **811** of the sheath **55,** and the second end **829** can be attached to the hub **56,** for example, to the engagement member **805.** In aspects, the plurality of wires **825** may extend through the lumen **815** of the sheath **55** between the engagement member **805** and the distal sheath end **811.** The plurality of wires **825** can comprise several types of wires. For example, in aspects, the plurality of wires **825** can comprise suture wires that can provide a pulling force (e.g., in a proximal direction **835**) but have limited to no ability to provide a pushing force (e.g., in a distal direction **837**). Alternatively, in aspects, the plurality of wires **825** can comprise wires of a higher stiffness that can provide both a pulling force and a pushing force. In aspects, when the plurality of wires **825** comprise a higher stiffness to provide both the pulling force and a pushing force, the sheath **55** may be biased (e.g., in a resting state in the absence of forces applied to the sheath **55**) toward the retracted position illustrated in **FIG. 9**, such that the plurality of wires **825** can apply a pushing force to the sheath **55** to move the sheath **55** from the retracted position illustrated in **FIG. 9** to the expanded position illustrated in **FIG. 8****.** Indeed, in aspects, the sheath **55** can comprise three possible states or diameters: (1) a first, or neutral state/position, wherein the sheath **55** has a diameter that is greater than the diameter **817** but less than the diameter **901;** (2) a second, or expanded, state/position, wherein the sheath **55** has the diameter **817** of **FIG. 8****;** and (3) a third, or retracted, state/position, wherein the sheath **55** has the diameter **901** of **FIG. 9****.** In such an example, the sheath **55** can be moved between the three states/diameters by applying a pushing force or pulling force via the plurality of wires **825.** In this way, the sheath **55** may be biased (e.g., in a resting state) toward the first, or neutral state/position, and may be moved to the second, or expanded, state/position by applying a pushing force to the sheath **55** via the wires **825,** and, conversely, may be moved to the third, or retracted, state/position by applying a pulling force to the sheath **55** via the wires **825.**

The engagement member **805** can move the plurality of wires **825** and the distal sheath end **811** between an extended position (e.g., illustrated in **FIG. 8**), in which the sheath **55** comprises the first cross-sectional dimension **817** and the first length **821,** and a retracted position (e.g., illustrated in **FIG. 9**), in which the sheath **55** comprises a second cross-sectional dimension **901** and a second length **903.** As described in the embodiments of **FIGS. 10-21****,** the engagement member **805** can move relative to the body portion **803,** for example, by rotating relative to the body portion **803,** moving axially relative to the body portion **803,** etc. which can cause the plurality of wires **825** to move in the proximal direction **835** or the distal direction **837.**

Referring to **FIG. 9****,** the engagement member **805** can move relative to the body portion **803** to cause the plurality of wires **825** to move in the proximal direction **835,** which can also cause the distal sheath end **811** to move in the proximal direction **835.** Methods can therefore comprise moving the plurality of wires **825** that are attached to the distal sheath end **811** of the sheath **55** in the proximal direction **835** toward the hub **56.** The movement of the engagement member **805** can increase the tension of the plurality of wires **825** and cause the plurality of wires **825** to move in the proximal direction **835.** Due to the attachment of the first end **827** of the plurality of wires **825** to the distal sheath end **811,** the distal sheath end **811** can move in the proximal direction **835,** which can increase the compression of the sheath **55.** This increase in compression of the sheath **55** can cause an increase of cross-sectional dimension (e.g., diameter) of the sheath **55,** from the first cross-sectional dimension **817** to the second cross-sectional dimension **901.** The second cross-sectional dimension **901** is greater than the first cross-sectional dimension **817,** such that when the distal sheath end **811** is moved in the proximal direction **835,** the sheath **55** can increase in cross-sectional size or diameter. The second length **903** is less than the first length **821,** such that when the distal sheath end **811** is moved in the proximal direction **835**, the length of the sheath **55** can decrease.

By controlling the cross-sectional size of the sheath **55** (e.g., via the plurality of wires **825**), the resistance forces that act upon passing structures (e.g., delivery device **75**, prosthesis **10**, etc.) that pass through the lumen **815** of the sheath **55** can be reduced. That is, when passing structures pass through the lumen **815,** contact between the passing structures and the sheath **55** may tend to increase tension to be applied to the sheath **55,** which causes constriction of the sheath **55** and increases friction between the sheath **55** and the passing structures. However, by increasing the cross-sectional size of the sheath **55** by applying the pulling force to the distal sheath end **811,** the likelihood of contact between the passing structures and an inner radial surface of the sheath **55** is decreased and/or the amount of friction between the passing structures and the inner radial surface of the sheath **55** is decreased. In the absence of tension applied to the distal sheath end **811** by the plurality of wires **825,** the sheath **55** can revert back to the position and dimensions illustrated in **FIG. 8****,** thus not restricting blood flow through the vessel **40.** In addition, or in the alternative, the plurality of wires **825** can comprise a stiffer material that can apply a pushing force (e.g., in the distal direction **837**) to facilitate movement of the sheath **55** to the position and dimensions illustrated in **FIG. 8****.** Accordingly, when no passing structures are within the lumen **815**, the sheath **55** may be at the smaller, first cross-sectional dimension **817,** but when passing structures are within the lumen **815,** the sheath **55** may expand to the larger, second cross-sectional dimension **817.** In aspects, the sheath **55** can comprise several materials, such as a Nitinol mesh. In aspects, the first cross-sectional dimension **817** may be within a range from about 12 Fr (e.g., French gauge) to about 20 Fr, and the second cross-sectional dimension **901** may be within a range from about 20 Fr to about 26 Fr. In one example, the first cross-sectional dimension **817** is about 18 Fr and the second cross-sectional dimension **901** is about 24 Fr. However, these dimensions are merely examples, and other possible dimensions are envisioned.

**FIGS. 10-13** illustrate an embodiment of the hub **56** comprising the body portion **803** and the engagement member **805.** For example, **FIG. 10** illustrates a side view of the hub **56** in engagement with the plurality of wires **825.** For purposes of illustration, the sheath **55** is not illustrated in **FIGS. 10-11** so as to not obscure view of the plurality of wires **825.** However, in operation, the sheath **55** is attached to the hub **56** as illustrated in **FIGS. 8-9** and **FIGS. 12-13****.** As illustrated in **FIG. 10****,** the engagement member **805** can be positioned within a recess **1001** of the hub **56** that is bordered on opposing axial sides by the body portion **803.** The engagement member **805** can therefore be attached to the body portion **803**, with the engagement member **805** configured to move relative to the body portion **803.** In this way, by being attached, the engagement member **805** can be connected to the body portion **803** (e.g., by being received within the recess **1001**) while not being limited to a single fixed position relative to the body portion **803.** Rather, in aspects, the engagement member **805** can rotate relative to the body portion **803,** for example, about a hub axis **1003** along which the hub **56** extends. The body portion **803** and the engagement member **805** can circumferentially surround the hub axis **1003** and the central passage **801** while extending coaxially with one another.

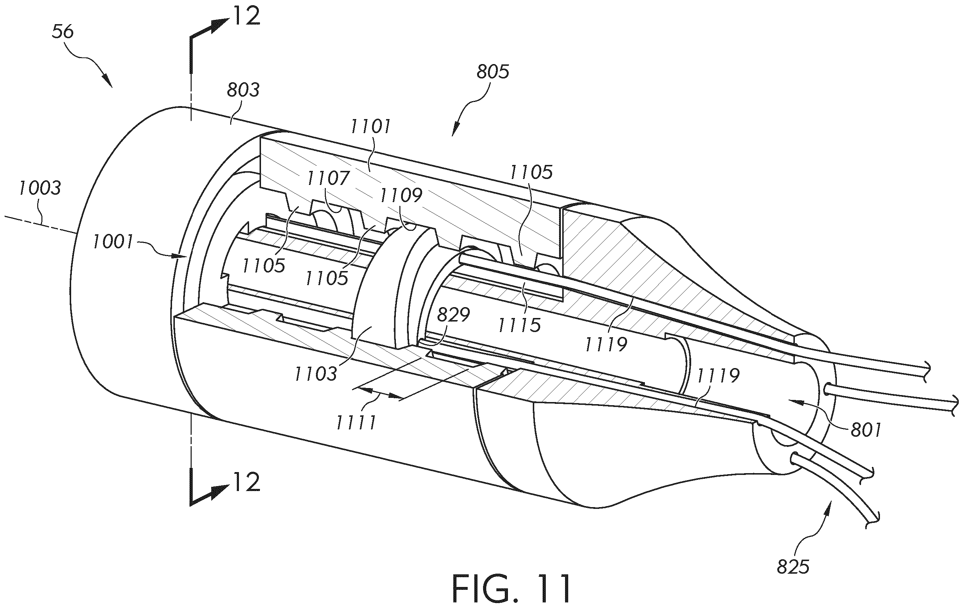

**FIG. 11** illustrates a partial sectional view of the hub **56** with a segment of the body portion **803** and the engagement member **805** removed in order to illustrate additional features of the hub **56,** wherein the segment is parallel to the hub axis **1003.** In aspects, the engagement member **805** can comprise a plurality of portions that can move relative to the body portion **803.** For example, the engagement member **805** can comprise a first engagement portion **1101** and a second engagement portion **1103.** The first engagement portion **1101** may circumferentially surround the second engagement portion **1103,** such that the second engagement portion **1103** is received within the first engagement portion **1101.** The first engagement portion **1101** is therefore positioned within the recess **1001,** with the first engagement portion **1101** comprising a length (e.g., along the hub axis **1003**) that substantially matches, or is slightly less than, the length of the recess **1001.** In aspects, the body portion **803** and the first engagement portion **1101** can comprise substantially matching cross-sectional sizes (e.g., diameters in **FIGS. 10-11** due to the substantially cylindrical shapes of the body portion **803** and the first engagement portion **1101**), though, differing cross-sectional sizes are possible. Accordingly, the first engagement portion **1101** is rotatable relative to the body portion **803,** with the first engagement portion **1101** circumferentially surrounding the central passage **801.**

In aspects, the first engagement portion **1101** can comprise one or more threads **1105** positioned on an inner radial side **1107** of the first engagement portion **1101.** The threads **1105** can wind helically around the hub axis **1003** on the inner radial side **1107** while extending at least partially along a length of the first engagement portion **1101.** The threads **1105** can be spaced axially apart along the hub axis **1003** to define a channel **1109** that winds helically around the hub axis **1003** between threads **1105.** The threads **1105** can project from an inner radial surface of the first engagement portion **1101** radially inwardly toward the hub axis **1003.** In this way, the inner radial surface is spaced a non-constant radial distance from the hub axis **1003** along a length of the first engagement portion **1101** due to the presence of the threads **1105** and the channel **1109.** The threads **1105** can facilitate the conversion of rotational movement (e.g., from rotation of the first engagement portion **1101**) to linear movement (e.g., linear movement of the second engagement portion **1103**).

The engagement member **805** can comprise the second engagement portion **1103** that is received within the first engagement portion **1101.** The second engagement portion **1103** can move axially relative to the first engagement portion **1101.** For example, in aspects, the second engagement portion **1103** can comprise a length **1111** that is less than the distance between neighboring threads **1105**, such that the second engagement portion **1103** can be positioned within the channel **1109.** In aspects, the second engagement portion **1103** can be positioned adjacent to one or more detent structures **1115** of the body portion **803** that limit rotation of the second engagement portion **1103** about the hub axis **1003.** For example, the one or more detent structures **1115** can comprise a ledge, outcropping, protuberance, extension, or the like that is adjacent to, and in contact with, circumferential ends of the second engagement portion **1103.** In aspects, a first circumferential end of the second engagement portion **1103** may be in contact with one detent structure **1115**, while an opposing second circumferential end of the second engagement portion **1103** may be in contact with another detent structure **1115.** In this way, the second engagement portion **1103** is limited from rotating due to being positioned circumferentially between detent structures **1115.** However, in aspects, other ways of limiting rotation of the second engagement portion **1103** can be provided, such that the hub **56** is not limited to the detent structures **1115.**

In aspects, the second end **829** of the plurality of wires **825** can be attached to the second engagement portion **1103** of the engagement member **805,** with the plurality of wires **825** extending through the central passage **801** of the hub **56.** The second end **829** of the plurality of wires **825** can be attached in any number of ways, for example, by adhesives, passing through channels of the second engagement portion **1103,** knots or other fastening mechanisms, etc. By being attached, movement of the second engagement portion **1103** can cause corresponding movement of the plurality of wires **825.** Though not required, in aspects, the central passage **801** of the body portion **803** can comprise one or more discrete wire openings **1119** through which the plurality of wires **825** can extend, with the one or more wire openings **1119** extending along the central axis **813** and the hub axis **1003.**

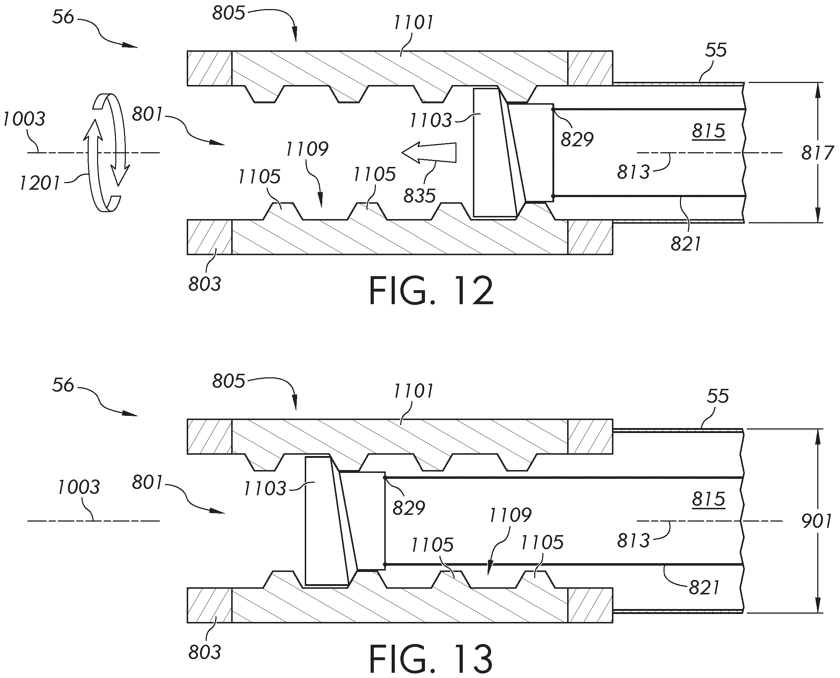

**FIGS. 12-13** illustrate a cross-sectional view of the hub **56** along lines **12-12** of **FIG. 11****.** For example, **FIG. 12** illustrates a position of the second engagement portion **1103** when the second engagement portion **1103** and the sheath **55** are in an extended position, which corresponds to the extended position illustrated in **FIG. 8****.** As illustrated in **FIG. 12****,** the second engagement portion **1103** is received within the channel **1109** between threads **1105.** Methods can comprise moving the plurality of wires **825** by rotating (e.g., illustrated schematically with arrowheads **1201**) the first engagement portion **1101** relative to the body portion 80**3** of the hub **56** such that the second engagement portion **1103**, which is received within the first engagement portion **1101,** moves axially relative to the first engagement portion **1101.** For example, as the first engagement portion **1101** rotates, the second engagement portion **1103** can move axially in a movement direction (e.g., the proximal direction **835**) along the hub axis **1003.** The detent structures **1115** of **FIG. 11** can limit the second engagement portion **1103** from rotating, such that the second engagement portion **1103** is limited to moving axially in response to rotation of the first engagement portion **1101**.

Referring to **FIG. 13**, the first engagement portion **1101** can continue to rotate **1201** at least until the second engagement portion **1103** and the sheath **55** are in a retracted position, which corresponds to the retracted position illustrated in **FIG. 9****.** As illustrated in **FIG. 13****,** rotation of the first engagement portion **1101** can move the second engagement portion **1103** axially in the proximal direction **835** along the hub axis **1003.** This proximal movement of the second engagement portion **1103** can likewise cause the plurality of wires **825** to move in the proximal direction **835.** Accordingly, axial movement (e.g., along the axes **813, 1003**) of the second engagement portion **1103** can cause the plurality of wires **825** and the distal sheath end **811** of the sheath **55** to move in the proximal direction **835.** As such, the sheath **55** can move from the extended position, comprising the first cross-sectional dimension **817** and the first length **821** (e.g., illustrated in **FIG. 8** and **FIG. 12**), to the retracted position, comprising the second cross-sectional dimension **901** and the second length **903** (e.g., illustrated in **FIG. 9** and **FIG. 13**).

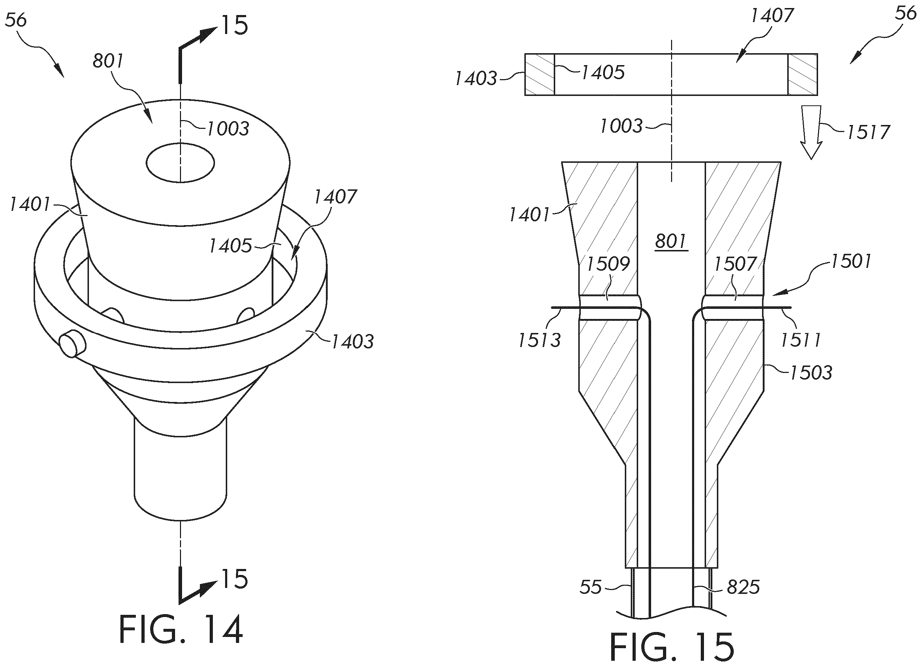

**FIGS. 14-18** illustrate another embodiment of the hub **56** comprising a body portion **1401** and an engagement member **1403.** For purposes of illustration, the sheath **55** is not illustrated in **FIG. 14** so as to not obscure view of the plurality of wires **825.** However, in operation, the sheath **55** is attached to the hub **56** as illustrated in **FIGS. 8-9** and **FIGS. 15-16****.** As illustrated in **FIG. 14****,** the engagement member **1403** can be positioned to circumferentially surround the body portion **1401.** The engagement member **1403** can be attached to the body portion **1401,** with the engagement member **1403** configured to move relative to the body portion **1401.** In this way, by being attached, the engagement member **1403** can be connected to the body portion **1401** (e.g., by circumferentially surrounding the body portion **1401**) while not being limited to a single fixed position relative to the body portion **1401**. Rather, in aspects, the engagement member **1403** can rotate relative to the body portion **1401**, for example, about the hub axis **1003** along which the hub **56** extends. The body portion **1401** and the engagement member **1403** can circumferentially surround the hub axis **1003** and the central passage **801** while extending coaxially with one another. The engagement member **1403** can comprise an inner radial surface **1405** that surrounds a recess **1407** within which the body portion **1401** is received.

**FIG. 15** is a sectional illustration of the hub **56** along lines **15-15** of **FIG. 14**, in which the engagement member **1403** is spaced apart from the body portion **1401** for the purposes of illustration and to more clearly show portions of the body portion **1401.** The body portion **1401** can define and circumferentially surround the central passage **801,** which may extend through the body portion **1401** along the hub axis **1003.** In aspects, the body portion **1401** can define at least one wire opening **1501** extending radially through the body portion **1401** between the central passage **801** and an outer radial side **1503** of the body portion **1401.** The at least one wire opening **1501** can extend through a wall of the body portion **1401** to define a passageway, path, etc., between the central passage **801** and an exterior of the body portion **1401** at the outer radial side **1503.** In aspects, the at least one wire opening **1501** may extend along a radial axis that is substantially perpendicular to the hub axis **1003**, however, any number of angles or orientations for the one wire opening **1501** can be provided. While **FIG. 15** illustrates the at least one wire opening **1501** comprising two wire openings (e.g., a first wire opening **1507** and a second wire opening **1509**) that are spaced circumferentially apart about 180 degrees about the hub axis **1003**, more than two wire openings can be provided. The at least one wire opening **1501** can define a path through which the plurality of wires **825** can extend from the central passage **801** to an exterior of the body portion **1401.** For example, a first wire **1511** of the plurality of wires **825** can extend through the first wire opening **1507,** and a second wire **1513** of the plurality of wires **825** can extend through the second wire opening **1509.** While **FIG. 15** illustrates one wire passing through one wire opening, the wire openings may be sized to accommodate any number (e.g., one or more) of wires of the plurality of wires **825.** In this way, one or more wires can pass through one wire opening.

The engagement member **1403** is substantially hollow, with the inner radial surface **1405** circumferentially surrounding the recess **1407.** In aspects, the body portion **1401** can comprise a cross-sectional size (e.g., diameter) that is less than a cross-sectional size (e.g., diameter) of the recess **1407.** In this way, the engagement member **1403** can be moved in a movement direction **1517** toward the body portion **1401**, such that the body portion **1401** can pass through, and be received within, the recess **1407.** In addition, or in the alternative, the body portion **1401** can be moved in a direction opposite the movement direction **1517** to cause the body portion **1401** to be through, and be received within, the recess **1407.**

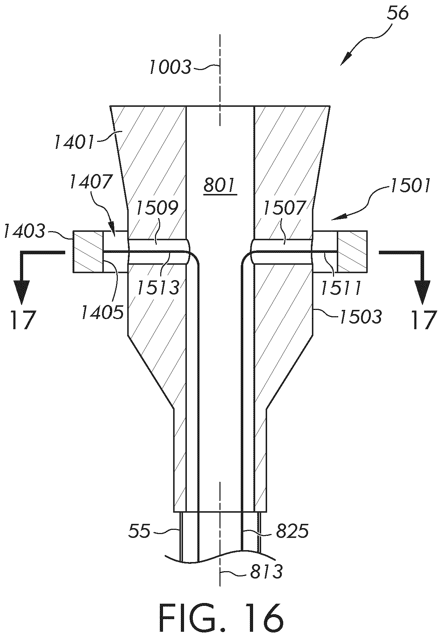

**FIG. 16** is a sectional illustration similar to **FIG. 15**, but for the engagement member **1403** receiving the body portion **1401**, and the plurality of wires **825** attached to the engagement member **1403.** For example, the engagement member **1403** can be positioned at an axial location along the hub axis **1003** that substantially matches a position of the wire openings **1501.** The diameter of the recess **1407** may be large enough such that the engagement member **1403** can rotate relative to the body portion **1401.** In aspects, the first wire **1511** can extend from the central passage **801**, through the first wire opening **1507**, and into the recess **1407**, whereupon the first wire **1511** is attached to the engagement member **1403.** In aspects, the first wire **1511** may be attached at an end (e.g., second end **829**) to a surface of the engagement member **1403**, such as, for example, the inner radial surface **1405.** Likewise, in aspects, the second wire **1513** can extend from the central passage **801,** through the second wire opening **1509,** and into the recess **1407**, whereupon the second wire **1513** is attached to the engagement member **1403.** In aspects, the second wire **1513** may be attached at an end (e.g., second end **829**) to a surface of the engagement member **1403**, such as, for example, the inner radial surface **1405.** Accordingly, the plurality of wires **825** can extend through the at least one wire opening **1501** and may be attached to the engagement member **1403.** The plurality of wires **825** can be attached in any number of ways to the engagement member **1403**, for example, by adhesives, knots or other fastening mechanisms, etc.

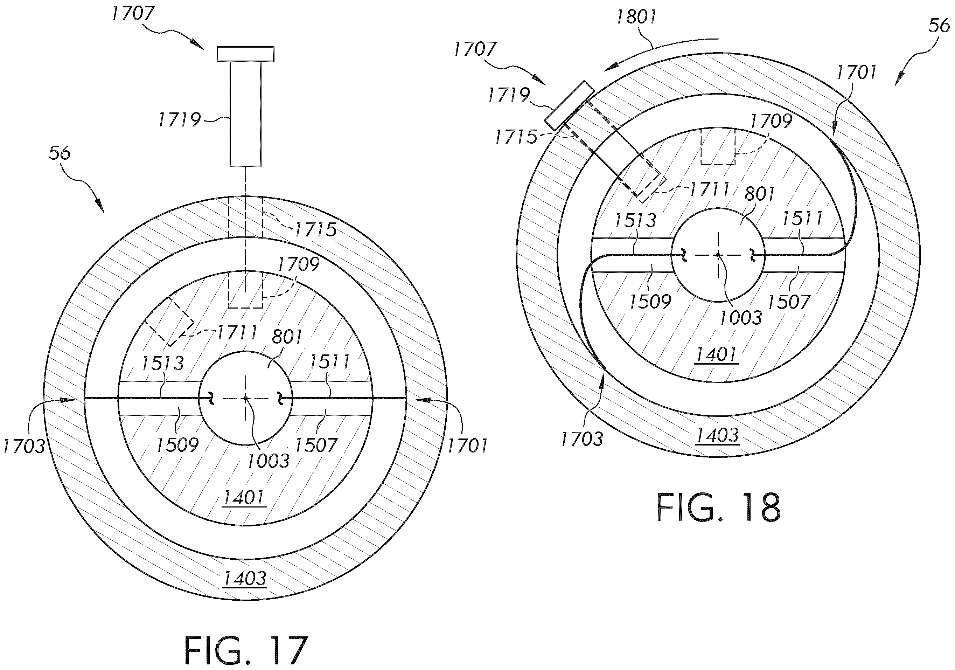

**FIG. 17** illustrates a top-down sectional illustration of the hub **56** along lines **17-17** of **FIG. 16**, in which the wires **1511**, **1513** extend from the central passage **801**, through the wire openings **1507**, **1509**, and to the engagement member **1403.** For example, **FIG. 17** illustrates a position of the engagement member **1403** when the engagement member **1403** and the sheath **55** are in an extended position, which corresponds to the extended position illustrated in **FIG. 8****.** In aspects, the first wire **1511** can be attached to the engagement member **1403** at a first attachment location **1701** of the engagement member **1403.** The second wire **1513** can be attached to the engagement member **1403** at a second attachment location **1703** of the engagement member **1403.** While **FIG. 17** illustrates the engagement member **1403** as comprising two attachment locations **1701**, **1703** that are spaced circumferentially apart about 180 degrees, any number of attachment locations (e.g., one or more) can be provided at varying locations along the engagement member **1403.** Further, while **FIG. 17** illustrates one wire attached to one attachment location, such a design is not intended to be limiting, as multiple wires can be attached to one attachment location.

Referring to **FIG. 18**, methods can comprise moving the plurality of wires **825** by rotating (e.g., illustrated with arrowhead **1801**) the engagement member **1403** relative to the body portion **1401** such that the plurality of wires **825** and the distal sheath end **811** of the sheath **55** move axially in the proximal direction **835**. The engagement member **1403** can be rotated **1801** at least until the engagement member **1403** and the sheath **55** are in a retracted position, which corresponds to the retracted position illustrated in **FIG. 9****.** As illustrated in **FIG. 18**, rotation of the engagement member **1403** can cause the first attachment location **1701** and the second attachment location **1703** to move and rotate relative to the body portion **1401** about the hub axis **1003.** In this way, the distance between the first attachment location **1701** and the first wire opening **1507** may increase as compared to the pre-rotation position illustrated in **FIG. 17****.** Likewise, the distance between the second attachment location **1703** and the second wire opening **1509** may increase as compared to the pre-rotation position illustrated in **FIG. 17****.** Due to the rotation of the first attachment location **1701** relative to the body portion **1401,** the length of the portion of the first wire **1511** extending between the first wire opening **1507** and the first attachment location **1701** can increase. Similarly, due to the rotation of the second attachment location **1703** relative to the body portion **1401,** the length of the portion of the second wire **1513** extending between the second wire opening **1509** and the second attachment location **1703** can also increase. As a result, the rotation of the engagement member **1403** can cause the plurality of wires **825** to move in the proximal direction **835** through the central passage **801**, which can cause the distal sheath end **811** of the sheath **55** to move in the proximal direction **835.** As such, the sheath **55** can move from the extended position, comprising the first cross-sectional dimension **817** and the first length **821** (e.g., illustrated in **FIG. 8**, which corresponds to the position illustrated in **FIG. 17**), to the retracted position, comprising the second cross-sectional dimension **901** and the second length **903** (e.g., illustrated in **FIG. 9** which corresponds to the position illustrated in **FIG. 18**).

In aspects, and as illustrated in **FIGS. 17-18****,** the hub **56** can comprise a locking mechanism **1707** that can selectively lock the engagement member **1403** relative to the body portion **1401** and prevent inadvertent or unintended rotation of the engagement member **1403** relative to the body portion **1401.** For example, referring to **FIG. 17**, the locking mechanism **1707** can comprise one or more openings in the body portion **1401**, for example, a first locking opening **1709**, a second locking opening **1711**, etc. The locking openings **1709**, **1711** may be formed at an outer radial side of the body portion **1401.** **FIG. 17** illustrates two locking openings **1709**, **1711** for the purposes of illustration, however, in aspects, additional locking openings (e.g., one or more) may be provided at the outer radial side of the body portion **1401** at several different locations. The locking mechanism **1707** can further comprise an engagement opening **1715** that extends radially through the engagement member **1403.**

In aspects, the locking mechanism **1707** can comprise a locking structure **1719** that is sized and shaped to be received within the locking openings **1709**, **1711** and the engagement opening **1715.** For example, the locking structure **1719** can comprise a screw, a bolt, a detent pin, etc. The locking structure **1719** can pass through the engagement opening **1715** and may be received within one of the first locking opening **1709** or the second locking opening **1711.** In aspects, to facilitate attachment of the locking structure **1719**, one or more of the openings **1709**, **1711**, **1715** may be threaded and/or the locking structure **1719** may be threaded. In this way, the locking structure **1719** can be removably received within the one or more of the openings **1709**, **1711**, **1715** without being inadvertently removed. As illustrated in **FIG. 18**, in aspects, after rotation of the engagement member **1403**, the locking structure **1719** can pass through the engagement opening **1715** and may be received within a locking opening, for example, the second locking opening **1711.** As such, the locking mechanism **1707** can limit unintended rotation of the engagement member **1403** relative to the body portion **1401.** The locking mechanism **1707** is not limited to the structure illustrated in **FIGS. 17-18****.** Rather, in the alternative, the locking mechanism **1707** can comprise other structures or configurations that can selectively prevent rotation of the engagement member **1403** relative to the body portion **1401.** For example, the locking mechanism **1707** can comprise a spring-loaded pin or locking structure **1719** housed in the body portion **1401** and projecting radially outwardly toward the engagement member **1403**, wherein the spring-loaded pin can be received within the engagement opening **1715.** It will be appreciated that while the locking mechanism **1707** is illustrated with respect to the embodiments of **FIGS. 14-18**, none, some, or all, of the other embodiments may comprise a locking mechanism that may be similar or identical to the locking mechanism **1707**, or different than the locking mechanism **1707.** In general, when the other embodiments comprise a locking mechanism, the locking mechanism can lock the engagement member relative to the body portion such that the sheath **55** can be held or locked in the extended position or retracted position (e.g., illustrated in **FIGS. 8-9**).

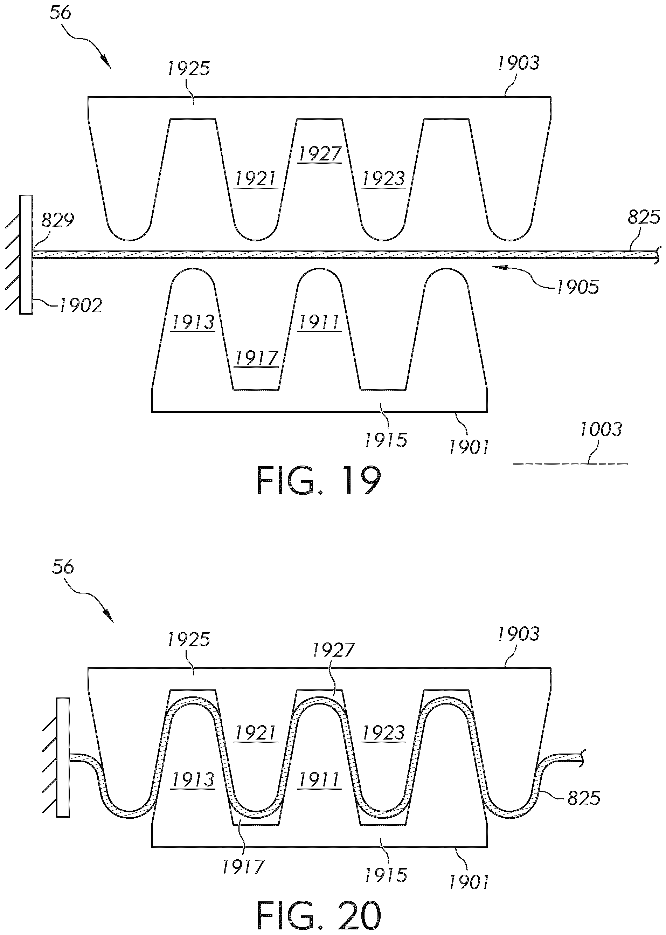

**FIGS. 19-20** illustrate another embodiment of the hub **56** comprising a body portion **1901** and an engagement member **1903.** For purposes of illustration, the sheath **55** is not illustrated in **FIG. 19** so as to not obscure view of the plurality of wires **825.** However, in operation, the sheath **55** is attached to the hub **56** as illustrated in **FIGS. 8-9****.** As illustrated in **FIG. 19**, the body portion **1901** and the engagement member **1903** can be in a spaced-apart orientation, with the plurality of wires **825** extending through a gap **1905** defined between the body portion **1901** and the engagement member **1903.** **FIG. 19** illustrates a position of the engagement member **1903** relative to the body portion **1901** when the engagement member **1903** and the sheath **55** are in an extended position, which corresponds to the extended position illustrated in **FIG. 8****.** As illustrated in **FIG. 19**, when the body portion **1901** and the engagement member **1903** are in the spaced-apart orientation, the plurality of wires **825** can extend along a linear travel path through the gap **1905.** The second end **829** of the plurality of wires **825** can be attached to a fixed structure **1902** (e.g., a portion of the body portion **1901**, etc.) that maintains a position of the plurality of wires **825** within the gap **1905.**

In aspects, the body portion **1901** can comprise one or more body projections, such as, for example, a first body projection **1911**, a second body projection **1913**, etc. The body projections **1911**, **1913** can project outwardly from a wall **1915** of the body portion **1901** and may project toward the engagement member **1903.** The body projections **1911**, **1913** can be spaced apart with a first body channel **1917** defined between the first body projection **1911** and the second body projection **1913.** In aspects, the body projections **1911**, **1913** can comprise a peak-like shape that each converge toward central points in a direction away from the wall **1915.** That is, adjacent to the wall **1915**, the body projections **1911**, **1913** comprise a maximum cross-sectional dimension, and may decrease in cross-sectional dimension in a direction away from the wall **1915.** While the body portion **1901** is illustrated as comprising three body projections and two body channels in **FIG. 19**, any number of body projections and body channels may be provided. The body projections are spaced apart with a body channel defined between two neighboring body projections, such that the body portion **1901** comprises alternating body projections and body channels along the wall **1915.**

In aspects, the engagement member **1903** can comprise one or more engagement projections, such as, for example, a first engagement projection **1921**, a second engagement projection **1923**, etc. The engagement projections **1921**, **1923** can project outwardly from a wall **1925** of the engagement member **1903** and may project toward the body portion **1901.** The engagement projections **1921, 1923** can be spaced apart with a first engagement channel **1927** defined between the first engagement projection **1921** and the second engagement projection **1923.** In aspects, the engagement projections **1921**, **1923** can comprise a peak-like shape that converges toward a central point in a direction away from the wall **1925.** That is, adjacent to the wall **1925**, the engagement projections **1921, 1923** comprise a maximum cross-sectional dimension, and may decrease in cross-sectional dimension in a direction away from the wall **1925.** While the engagement member **1903** is illustrated as comprising four engagement projections and three engagement channels in **FIG. 19**, any number of engagement projections and engagement channels may be provided. The engagement projections may be spaced apart with an engagement channel defined between two neighboring engagement projections, such that the engagement member **1903** comprises alternating engagement projections and engagement channels along the wall **1925.** In aspects, the size and shape of the engagement projections **1921, 1923** may substantially match a size and shape of the body projections **1911, 1913,** and the size and shape of the body channels **1917** can substantially match a size and shape of the engagement channels **1927.** In aspects, other shapes of the body projections **1911, 1913** and the engagement projections **1921, 1923** can be provided, for example, with the body projections **1911**, **1913** comprising steps, teeth, or ridges that match and engage with corresponding steps, teeth, or ridges of the and the engagement projections **1921, 1923.** In this way, additional control over the movement of the plurality of wires **825** can be provided.

In aspects, the body projections **1911, 1913** may be offset from the engagement projections **1921, 1923** along the hub axis **1003.** In this way, a body projection can be received within an engagement channel. Likewise, an engagement projection can be received within a body channel. For example, as illustrated in **FIG. 20**, methods can comprise moving the plurality of wires **825** by moving the engagement member **1903** and the body portion **1901** together to compress at least one wire **825** such that the at least one wire **825** extends along a non-linear travel path. Moving the engagement member **1903** and the body portion **1901** can comprise moving the engagement member **1903** relative to the body portion **1901** (e.g., by moving the engagement member **1903** toward the body portion **1901**), moving the body portion **1901** relative to the engagement member **1903** (e.g., by moving the body portion **1901** toward the engagement member **1903**), and/or moving both the engagement member **1903** and the body portion **1901** (e.g., by moving the engagement member **1903** toward the body portion **1901** concurrently with moving the body portion **1901** toward the engagement member **1903**). In this way, the body projections **1911,1913** can be received within engagement channels **1927,** and engagement projections **1921**, **1923** can be received within body channels **1917.** For example, the first body projection **1911** can be received within the first engagement channel **1927**, which can likewise cause one or more wires of the plurality of wires **825** to move from the linear path (e.g., illustrated in **FIG. 18**) to moving along a non-linear path and extending within the first engagement channel **1927.** Likewise, the first engagement projection **1921** can be received within the first body channel **1917**, which can likewise cause one or more wires of the plurality of wires **825** to move from the linear path (e.g., illustrated in **FIG. 18**) to moving along a non-linear path and extending within the first body channel **1917.** As illustrated herein, in aspects, the projections **1911**, **1913**, **1921**, **1923** can comprise a rounded or blunt shape or tip to reduce the likelihood of damage to the wires **825.** That is, the end of the projections **1911**, **1913**, **1921**, **1923** that contacts the wires **825** may have the rounded or blunt shape.

Accordingly, the engagement member **1903** and the body portion **1901** can move relative to one another between a first position (e.g., illustrated in **FIG. 18**) and a second position (e.g., illustrated in **FIG. 19**). In the first position, the engagement member **1903** is spaced apart from the body portion **1901** such that the first body projection **1911** is not received within the first engagement channel **1927** (e.g., or any engagement channel), and at least one wire of the plurality of wires **825** extends between the engagement member **1903** and the body portion **1901** (e.g., along a linear travel path and outside of the body channels or engagement channels). In the second position, the first body projection **1911** is received within the first engagement channel **1927**, and at least one wire of the plurality of wires **825** extends within the engagement channels **1927** and the body channels **1917** along the non-linear travel path. As a result, due to the plurality of wires **825** being contacted and moved by the body portion **1901** and the engagement member **1903**, the plurality of wires **825** can move in the proximal direction **835**, which can cause the distal sheath end **811** of the sheath **55** to move in the proximal direction **835.** As such, the sheath **55** can move from the extended position, comprising the first cross-sectional dimension **817** and the first length **821** (e.g., illustrated in **FIG.** 8, which corresponds to the position illustrated in **FIG. 19**), to the retracted position, comprising the second cross-sectional dimension **901** and the second length **903** (e.g., illustrated in **FIG. 9** which corresponds to the position illustrated in **FIG. 20**).

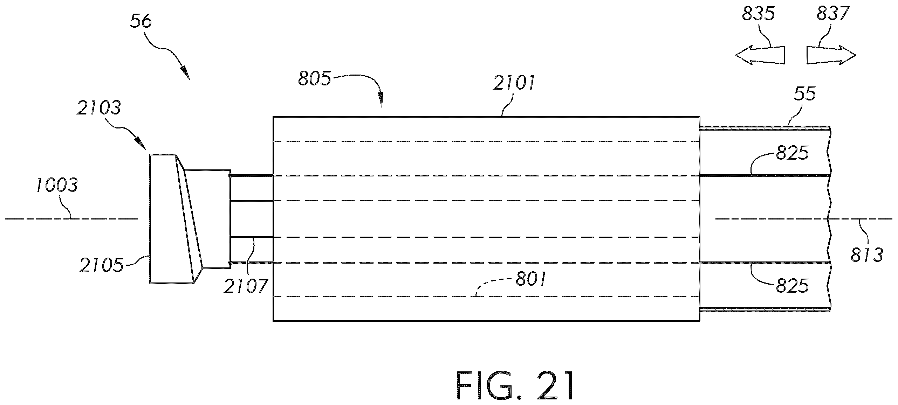

**FIG. 21** illustrates another embodiment of the hub **56**, wherein the hub **56** comprises a body portion **2101** and an engagement member **2103.** In aspects, the body portion **2101** can at least partially receive a portion of the engagement member **2103** within the central passage **801.** For example, the engagement member **2103** can comprise a handle **2105** and a slider **2107.** The handle **2105** and the slider **2107** can be attached to one another and may extend along the axis **1003.** In aspects, the slider **2107** can project outwardly from the handle **2105** and may be received within the central passage **801**, while the handle **2105** may be located at an exterior of the central passage **801.** In aspects, the plurality of wires **825** may be attached to one, or both, of the handle **2105** or the slider **2107.** In operation, a user can manipulate the handle **2105** and move the handle **2105** relative to the body portion **2101**, wherein movement of the handle **2105** can likewise cause movement of the slider **2107.** For example, the handle **2105** can be moved in the proximal direction **835.** Due to the plurality of wires **825** being attached to the engagement member **2103** (e.g., to one or more of the handle **2105** or the slider **2107**), the plurality of wires **825** can move in the proximal direction **835**, which can cause the distal sheath end **811** of the sheath **55** to move in the proximal direction **835.** As such, the sheath **55** can move from the extended position, comprising the first cross-sectional dimension **817** and the first length **821** (e.g., illustrated in **FIG. 8**), to the retracted position, comprising the second cross-sectional dimension **901** and the second length **903** (e.g., illustrated in **FIG. 9**).

Aspect 1. An introducer apparatus is provided for delivering a heart valve prosthesis to a treatment site. The introducer apparatus comprises a hub comprising a central passage and an engagement member. The introducer apparatus comprises a sheath attached to the hub and comprising a first cross-sectional dimension along a plane that is perpendicular to a central axis of the sheath. The introducer apparatus comprises a plurality of wires extending between a first end and an opposing second end. The first end is attached to a distal sheath end of the sheath and the second end is attached to the engagement member. The engagement member is configured to move the plurality of wires and the distal sheath end between an extended position, in which the sheath comprises the first cross-sectional dimension, and a retracted position, in which the sheath comprises a second cross-sectional dimension that is greater than the first cross-sectional dimension.

Aspect 2. The introducer apparatus of aspect 1, wherein the engagement member is attached to a body portion of the hub, the engagement member configured to move relative to the body portion.

Aspect 3. The introducer apparatus of any of aspects 1-2, wherein the engagement member comprises a first engagement portion that is rotatable relative to the body portion, the first engagement portion circumferentially surrounding the central passage; and a second engagement portion received within the first engagement portion, the second engagement portion configured to move axially relative to the first engagement portion.

Aspect 4. The introducer apparatus of any of aspects 1-3, wherein the second end of the plurality of wires is attached to the second engagement portion such that the plurality of wires extend along the sheath.

Aspect 5. The introducer apparatus of any of aspects 1-4, wherein the engagement member circumferentially surrounds the body portion.

Aspect 6. The introducer apparatus of any of aspects 1-5, wherein the body portion defines at least one wire opening extending radially through the body portion between the central passage and an outer radial side of the body portion, the plurality of wires extending through the at least one wire opening such that the second end is attached to the engagement member.

Aspect 7. The introducer apparatus of any of aspects 1-6, wherein the engagement member is configured to rotate relative to the body portion, and wherein a locking mechanism contacts the engagement member and the body portion to selectively prevent rotation of the engagement member relative to the body portion.

Aspect 8. The introducer apparatus of any of aspects 1-7, wherein the engagement member comprises a first engagement projection and a second engagement projection that are spaced apart to define a first engagement channel between the first engagement projection and the second engagement projection.

Aspect 9.The introducer apparatus of any of aspects 1-8, wherein the body portion comprises a first body projection that is configured to be received within the first engagement channel such that the engagement member and the body portion are configured to move relative to one another between: a first position, in which the engagement member is spaced apart from the body portion such that the first body projection is not received within the first engagement channel, and at least one wire of the plurality of wires extends between the engagement member and the body portion; and a second position, in which the first body projection is received within the first engagement channel, and at least one wire of the plurality of wires extends within the first engagement channel.

Aspect 10. An introducer apparatus is provided for delivering a heart valve prosthesis to a treatment site. The introducer apparatus comprises a hub comprising a body portion surrounding a central passage, and an engagement member attached to the body portion and configured to move relative to the body portion. The introducer apparatus comprises a sheath attached to the hub. The sheath comprises a lumen in communication with the central passage. The sheath comprises a first cross-sectional dimension along a plane that is perpendicular to a central axis of the sheath. A plurality of wires extend between a first end and an opposing second end. The first end is attached to a distal sheath end of the sheath and the second end is attached to the engagement member. The engagement member is configured to move the plurality of wires and the distal sheath end from an extended position, in which the sheath comprises the first cross-sectional dimension and a first length, to a retracted position, in which the sheath comprises a second cross-sectional dimension, which is greater than the first cross-sectional dimension, and a second length, which is less than the first length.

Aspect 11. The introducer apparatus of aspect 10, wherein the engagement member comprises: a first engagement portion that is rotatable relative to the body portion, the first engagement portion circumferentially surrounding the central passage; and a second engagement portion received within the first engagement portion, the second engagement portion configured to move axially relative to the first engagement portion, the second end of the plurality of wires attached to the second engagement portion such that the plurality of wires extend along the sheath.

Aspect 12. The introducer apparatus of any of aspects 10-11, wherein the engagement member circumferentially surrounds the body portion and is configured to rotate relative to the body portion, and wherein the body portion defines at least one wire opening extending radially through the body portion between the central passage and an outer radial side of the body portion, the plurality of wires extending through the at least one wire opening such that the second end is attached to the engagement member.

Aspect 13. The introducer apparatus of any of aspects 10-12, wherein the engagement member comprises a first engagement projection and a second engagement projection that are spaced apart to define a first engagement channel between the first engagement projection and the second engagement projection, and wherein the body portion comprises a first body projection that is configured to be received within the first engagement channel, at least one wire of the plurality of wires extending between the engagement member and the body portion.

Aspect 14. Methods of delivering a heart valve prosthesis to a treatment site comprising providing a sheath attached to a hub, the sheath comprising a first cross-sectional dimension along a plane that is perpendicular to a central axis of the sheath. Methods comprise moving a plurality of wires that are attached to a distal sheath end of the sheath in a proximal direction toward the hub. Methods comprise increasing a cross-sectional dimension of the sheath.

Aspect 15. The method of aspect 14, wherein moving the plurality of wires comprises rotating a first engagement portion relative to a body portion of the hub such that a second engagement portion, which is received within the first engagement portion, moves axially relative to the first engagement portion.

Aspect 16. The method of any one of aspects 14-15, wherein the plurality of wires are attached to the second engagement portion such that the axial movement of the second engagement portion causes the plurality of wires and the distal sheath end of the sheath to move in the proximal direction.

Aspect 17. The method of any one of aspects 14-16, wherein the plurality of wires extend through at least one wire opening of a body portion of the hub and are attached to an engagement member of the hub.

Aspect 18. The method of any one of aspects 14-17, wherein moving the plurality of wires comprises rotating the engagement member relative to the body portion such that the plurality of wires and the distal sheath end of the sheath move axially in the proximal direction.

Aspect 19. The method of any one of aspects 14-18, wherein at least one wire of the plurality of wires is positioned between an engagement member of the hub and a body portion of the hub, the at least one wire extending along a linear travel path.

Aspect 20. The method of any one of aspects 14-19, wherein moving the plurality of wires comprises moving the engagement member and the body portion together to compress the at least one wire such that the at least one wire extends along a non-linear travel path.

Further disclosed herein is the subject matter of the following clauses:
1. An introducer apparatus for delivering a heart valve prosthesis (10) to a treatment site (701), the introducer apparatus comprising:
   a hub (56) comprising a central passage (801) and an engagement member (805);
   a sheath (55) attached to the hub (56) and comprising a first cross-sectional dimension (817) along a plane (819) that is perpendicular to a central axis (813) of the sheath (55); and
   a plurality of wires (825) extending between a first end (827) and an opposing second end (829), the first end (827) attached to a distal sheath end (811) of the sheath (55) and the second end (829) attached to the engagement member (805), the engagement member (805) configured to move the plurality of wires (825) and the distal sheath end (811) between:
      an extended position, in which the sheath (55) comprises the first cross-sectional dimension (817); and
      a retracted position, in which the sheath (55) comprises a second cross-sectional dimension that is greater than the first cross-sectional dimension (817).
2. The introducer apparatus of clause 1, wherein the engagement member (805) is attached to a body portion (803) of the hub (56), the engagement member (805) configured to move relative to the body portion (803).
3. The introducer apparatus of clause 2, wherein the engagement member (805) comprises:
   a first engagement portion (1101) that is rotatable relative to the body portion (803), the first engagement portion (1101) circumferentially surrounding the central passage (801); and
   a second engagement portion (1103) received within the first engagement portion (1101), the second engagement portion (1103) configured to move axially relative to the first engagement portion (1101).
4. The introducer apparatus of clause 3, wherein the second end (829) of the plurality of wires (825) are attached to the second engagement portion (1103) such that the plurality of wires (825) extend along the sheath (55).
5. The introducer apparatus of clause 2, wherein the engagement member (1403) circumferentially surrounds the body portion (1401).
6. The introducer apparatus of clause 5, wherein the body portion (1401) defines at least one wire opening extending radially through the body portion (1401) between the central passage (801) and an outer radial side of the body portion (1401), the plurality of wires (825) extending through the at least one wire opening such that the second end (829) is attached to the engagement member (1403).
7. The introducer apparatus of clause 6, wherein the engagement member (1403) is configured to rotate relative to the body portion (1401), and wherein a locking mechanism (1707) contacts the engagement member (1403) and the body portion (1401) to selectively prevent rotation of the engagement member (1403) relative to the body portion (1401).
8. The introducer apparatus of clause 2, wherein the engagement member (1903) comprises a first engagement projection (1921) and a second engagement projection (1923) that are spaced apart to define a first engagement channel (1927) between the first engagement projection (1921) and the second engagement projection (1923).
9. The introducer apparatus of clause 8, wherein the body portion (1901) comprises a first body projection (1911) that is configured to be received within the first engagement channel (1927) such that the engagement member (1903) and the body portion (1901) are configured to move relative to one another between:
   a first position, in which the engagement member (1903) is spaced apart from the body portion (1901) such that the first body projection (1911) is not received within the first engagement channel (1927), and at least one wire of the plurality of wires (825) extends between the engagement member (1903) and the body portion (1901); and
   a second position, in which the first body projection (1911) is received within the first engagement channel (1927), and at least one wire of the plurality of wires (825) extends within the first engagement channel (1927).

Further disclosed herein is an introducer apparatus provided for delivering a heart valve prosthesis to a treatment site. The introducer apparatus includes a hub including a central passage and an engagement member. The introducer apparatus includes a sheath attached to the hub and including a first cross-sectional dimension. A plurality of wires extend between a first end and an opposing second end. The first end is attached to a distal sheath end of the sheath and the second end is attached to the engagement member. The engagement member moves the plurality of wires and the distal sheath end between an extended position, in which the sheath includes the first cross-sectional dimension, and a retracted position, in which the sheath includes a second cross-sectional dimension that is greater than the first cross-sectional dimension.

It should be understood that while various aspects have been described in detail relative to certain illustrative and specific examples thereof, the present disclosure should not be considered limited to such, as numerous modifications and combinations of the disclosed features are possible without departing from the scope of the following claims.

## Claims

1. An introducer apparatus for delivering a heart valve prosthesis (10) to a treatment site (701), the introducer apparatus comprising:
a hub (56) comprising a central passage (801) and an engagement member (805);
a sheath (55) attached to the hub (56) and comprising a first cross-sectional dimension (817) along a plane (819) that is perpendicular to a central axis (813) of the sheath (55); and
a plurality of wires (825) extending between a first end (827) and an opposing second end (829), the first end (827) attached to a distal sheath end (811) of the sheath (55) and the second end (829) attached to the engagement member (805), the engagement member (805) configured to move the plurality of wires (825) and the distal sheath end (811) between:
an extended position, in which the sheath (55) comprises the first cross-sectional dimension (817); and
a retracted position, in which the sheath (55) comprises a second cross-sectional dimension that is greater than the first cross-sectional dimension (817).

2. The introducer apparatus of claim 1, wherein the engagement member (805) is attached to a body portion (803) of the hub (56), the engagement member (805) configured to move relative to the body portion (803).

3. The introducer apparatus of claim 2, wherein the engagement member (805) comprises:
a first engagement portion (1101) that is rotatable relative to the body portion (803), the first engagement portion (1101) circumferentially surrounding the central passage (801); and
a second engagement portion (1103) received within the first engagement portion (1101), the second engagement portion (1103) configured to move axially relative to the first engagement portion (1101).

4. The introducer apparatus of claim 3, wherein the second end (829) of the plurality of wires (825) are attached to the second engagement portion (1103) such that the plurality of wires (825) extend along the sheath (55).

5. The introducer apparatus of any one of claims 2 to 4, wherein the engagement member (1403) circumferentially surrounds the body portion (1401).

6. The introducer apparatus of any one of claims 2 to 5, wherein the body portion (1401) defines at least one wire opening extending radially through the body portion (1401) between the central passage (801) and an outer radial side of the body portion (1401), the plurality of wires (825) extending through the at least one wire opening such that the second end (829) is attached to the engagement member (1403).

7. The introducer apparatus of any one of claims 2 to 6, wherein the engagement member (1403) is configured to rotate relative to the body portion (1401), and wherein a locking mechanism (1707) contacts the engagement member (1403) and the body portion (1401) to selectively prevent rotation of the engagement member (1403) relative to the body portion (1401).

8. The introducer apparatus of any one of the preceding claims, wherein the engagement member (1903) comprises a first engagement projection (1921) and a second engagement projection (1923) that are spaced apart to define a first engagement channel (1927) between the first engagement projection (1921) and the second engagement projection (1923).

9. The introducer apparatus of claim 8, wherein the body portion (1901) comprises a first body projection (1911) that is configured to be received within the first engagement channel (1927) such that the engagement member (1903) and the body portion (1901) are configured to move relative to one another between:
a first position, in which the engagement member (1903) is spaced apart from the body portion (1901) such that the first body projection (1911) is not received within the first engagement channel (1927), and at least one wire of the plurality of wires (825) extends between the engagement member (1903) and the body portion (1901); and
a second position, in which the first body projection (1911) is received within the first engagement channel (1927), and at least one wire of the plurality of wires (825) extends within the first engagement channel (1927).
